# EUROPÄISCHE PATENTSCHRIFT

(11) **EP 2 907 470 B1**
(45) Veröffentlichungstag und Bekanntmachung des Hinweises auf die Patenterteilung: **02.05.2018**
(21) Anmeldenummer: 15155081.1
(22) Anmeldetag: 13.02.2015
(51) Int. Cl.: A61B 50/10, A47B 57/40

(54) **Gerätewagen**
Device trolley
Servante d'outillage

(30) Priorität: 18.02.2014 DE 102014101999
(43) Veröffentlichungstag der Anmeldung: 19.08.2015
(73) Patentinhaber: Karl Storz SE & Co. KG, 78532 Tuttlingen (DE)
(72) Erfinder: Sabo, Alexander, 78532 Tuttlingen (DE); Jansche, Andreas, 78532 Tuttlingen (DE); Gümpel, Anika, 78532 Tuttlingen (DE); Müller-Beiter, Wolfgang, 72517 Sigmaringendorf (DE); Schairer, Rainer, 72459 Albstadt (DE)

(56) Entgegenhaltungen:
- EP-A1- 1 238 884
- WO-A1-2007/077609
- DE-A1- 2 856 830
- DE-A1-102007 053 327
- DE-U1-202010 007 467
- US-A- 4 406 374
- US-A- 5 022 621
- US-A1- 2004 108 427
- US-A1- 2013 146 551

## Beschreibung

Die vorliegende Erfindung ist auf einen Gerätewagen oder einen anderen Geräteträger und auf eine Trageinrichtung zur Anbringung an einem Geräteträger und zum Tragen eines medizinischen Geräts, eines medizinischen Instruments oder einer anderen Nutzlast in einem medizinischen Behandlungsraum gerichtet.

In Operationssälen und anderen medizinischen Behandlungsräumen kommen oft viele medizinische Geräte und Instrumente gleichzeitig oder nacheinander zum Einsatz. Ein medizinischer Geräteträger, insbesondere ein medizinischer Gerätewagen, d.h. ein medizinischer Geräteträger, der auf Rädern verfahrbar ist, kann eine ökonomisch und ergonomisch günstige und flexible Anordnung einer Vielzahl von medizinischen Geräten und Instrumenten ermöglichen.

Ein medizinischer Gerätewagen oder ein anderer medizinischer Geräteträger soll nicht nur robust sein und eine einfache Reinigung und Sterilisation ermöglichen, sondern auch mit geringem Aufwand modifiziert bzw. verändert (insbesondere erweitert) und, möglichst auch von Personen ohne technische Ausbildung sicher und fehlerfrei zusammengesetzt werden können. Ein medizinischer Geräteträger soll insbesondere mit geringem Aufwand durch Austauschen oder Versetzen von Trageinrichtungen bzw. Ablageelementen an neue Aufgaben anpassbar sein. Insbesondere sollen Trageinrichtungen bzw. Ablageelemente einerseits ohne Werkzeug montiert und demontiert werden können und andererseits im montierten Zustand zuverlässig dauerhaft mit dem medizinischen Geräteträger verbunden sein.

Bestehende medizinische Geräteträger und - wagen sind aus der WO2007077609 A1, sowie der EP1238884 A1 bekannt. Nicht auf den medizinischen Bereich beschränkte Trageinrichtungen mit Verriegelungssystemen gemäß des Oberbegriffs von Anspruch 1 werden unter anderem durch die DE202010007467U, US2013146551 A1 und US4406374 A offenbart.

Eine Aufgabe der vorliegenden Erfindung besteht darin, eine verbesserte Trageinrichtung zur Anbringung an einem Geräteträger und zum Tragen eines medizinischen Geräts, eines medizinischen Instruments oder einer anderen Nutzlast und einen verbesserten medizinischen Geräteträger zu schaffen.

Diese Aufgabe wird durch den Gegenstand von Anspruch 1 gelöst. Weiterbildungen sind in den abhängigen Ansprüchen angegeben.

Eine Trageinrichtung zur lösbaren Verbindung mit einem Geräteträger und zum Tragen eines medizinischen Geräts, eines medizinischen Instruments oder einer anderen Nutzlast umfasst einen Eingreifhaken zum Eingreifen in eine Öffnung an einem Geräteträger und einen Riegel zum Verriegeln einer Verbindung zwischen dem Eingreifhaken und einer Öffnung, in die der Eingreifhaken eingreift.

Die Trageinrichtung ist insbesondere zur Anbringung an einem mobilen Geräteträger bzw. an einem Gerätewagen für medizinische oder nicht-medizinische Anwendungen oder an einem stationären Geräteträger für medizinische oder nicht-medizinische Anwendungen vorgesehen. Der Eingreifhaken ist insbesondere zum Eingreifen in eine von mehreren Öffnungen an einem Geräteträger vorgesehen, um die Trageinrichtung in einer von mehreren alternativen Positionen mit dem Geräteträger lösbar zu verbinden. Die Trageinrichtung kann einen oder mehrere weitere Eingreifhaken aufweisen, wobei an jedem Eingreifhaken ein Riegel zum Verriegeln einer Verbindung zwischen dem weiteren Eingreifhaken und einer Öffnung vorgesehen sein kann. Ferner kann die Trageinrichtung einen oder mehrere Stifte, Nasen oder andere Einrichtungen zum Abstützen der Trageinrichtung an weiteren Öffnungen oder Stufen an dem Geräteträger aufweisen.

Durch Einführen des Eingreifhakens in eine Öffnung an einem Geräteträger kann die Trageinrichtung schnell in einer oder in einer von mehreren alternativen Positionen an einem Geräteträger eingehängt werden. Der Riegel ermöglicht eine Verriegelung der Verbindung zwischen dem Eingreifhaken und einer Öffnung, in die der Eingreifhaken eingreift und ein späteres Entriegeln der Verbindung ohne Werkzeug. Die Trageinrichtung kann deshalb schnell und mit geringem Aufwand versetzt oder ausgetauscht werden, um einen Geräteträger zu modifizieren und an neue Aufgaben anzupassen.

Bei einer Trageinrichtung, wie sie hier beschrieben ist, weist der Eingreifhaken insbesondere einen geraden Abschnitt zum Eingreifen in eine Öffnung und einen gegenüber dem geraden Abschnitt abgewinkelten Abschnitt zum Hintergreifen eines Rands der Öffnung auf, wobei der Riegel parallel zu dem geraden Abschnitt des Eingreifhakens zwischen einer entriegelnden Position und einer verriegelnden Position verschiebbar ist.

Der Eingreifhaken weist also insbesondere eine L-förmige Gestalt auf. Bei der vorgesehenen Verwendung der Trageinrichtung sind insbesondere der gerade Abschnitt horizontal oder im Wesentlichen horizontal und der gegenüber dem geraden Abschnitt abgewinkelte Abschnitt vertikal oder im Wesentlichen vertikal angeordnet. Der gegenüber dem geraden Abschnitt abgewinkelte Abschnitt ist insbesondere zum Hintergreifen eines Stegs zwischen zwei Öffnungen in einer Reihe von Öffnungen vorgesehen und ausgebildet. Der Riegel ist in der verriegelnden Position insbesondere so angeordnet, dass er eine Bewegung des Eingreifhakens in einer Richtung orthogonal zum geraden Abschnitt bzw. parallel oder im Wesentlichen parallel zum abgewinkelten Abschnitt unterbindet. Damit kann der Riegel in seiner verriegelnden Position bewirken, dass der gegenüber dem geraden Abschnitt abgewinkelte Abschnitt des Eingreifhakens nicht aus seiner den Rand der Öffnung hintergreifenden Position herausbewegt werden kann.

Der Eingreifhaken und der Riegel sind insbesondere so ausgestaltet, dass der Eingreifhaken in der Öffnung an einem Geräteträger nur dann in einer Richtung orthogonal zu einem geraden Abschnitt des Eingreifhakens bewegt werden kann, wenn der Riegel in seiner entriegelnden Position ist. In der verriegelnden Position hält der Riegel den Eingreifhaken insbesondere in einer Position relativ zu der Öffnung, in der ein gegenüber dem geraden Abschnitt abgewinkelter Abschnitt des Eingreifhakens einen Rand der Öffnung hintergreift.

Bei einer Trageinrichtung, wie sie hier beschrieben ist, ist der Riegel insbesondere vorgesehen und ausgebildet, um in der entriegelnden Position nicht oder im Wesentlichen nicht neben dem geraden Abschnitt des Eingreifhakens angeordnet zu sein, und um in der verriegelnden Position neben dem geraden Abschnitt des Eingreifhakens angeordnet zu sein.

Der Riegel ist in seiner verriegelnden Position insbesondere - zumindest teilweise - innerhalb derselben Öffnung angeordnet, in der der Eingreifhaken, ggf. insbesondere dessen gerader Abschnitt, angeordnet ist. Alternativ kann der Riegel vorgesehen und angeordnet sein, um in seiner verriegelnden Position in eine andere, insbesondere in eine benachbarte Öffnung einzugreifen.

Bei einer Trageinrichtung, wie sie hier beschrieben ist, ist der Riegel insbesondere vorgesehen und ausgebildet, um in der entriegelnden Position nicht oder im Wesentlichen nicht in eine Öffnung an einem Geräteträger, in die der Eingreifhaken eingreift, einzugreifen, und um in der verriegelnden Position in eine Öffnung an einem Geräteträger, in die der Eingreifhaken eingreift, einzugreifen.

Eine Trageinrichtung, wie sie hier beschrieben ist, umfasst insbesondere ferner eine Sicherungseinrichtung zum Halten des Riegels in der verriegelnden Position.

Bei einer Trageinrichtung, wie sie hier beschrieben ist, ist die Sicherungseinrichtung insbesondere ausgebildet, um den Riegel rastend in der verriegelnden Position zu halten.

Darüber hinaus kann die Sicherungseinrichtung ausgebildet sein, um den Riegel auch in seiner entriegelnden Position durch elastisches Rasten oder auf andere Weise zu halten. Die Sicherungseinrichtung ist insbesondere so vorgesehen und ausgebildet, dass zum Verschieben des Riegels zwischen seiner verriegelnden Position und seiner entriegelnden Position (und optional auch in der Gegenrichtung) ein mechanischer Widerstand überwunden werden muss.

Die Sicherungseinrichtung umfasst beispielsweise eine oder mehrere Nocken am Riegel, die in eine oder mehrere Nuten an der Trageinrichtung eingreifen können, wenn der Riegel sich in der verriegelnden Position befindet. Alternativ können ein oder mehrere Nocken an der Trageinrichtung und ein oder mehrere Nuten am Riegel oder sowohl an der Trageinrichtung als auch am Riegel Nocken vorgesehen sein. Eine Elastizität des Nockens oder der Nocken oder der Nut oder der Nuten ermöglicht, dass die Nocken und Nut bei Überwindung der elastischen Gegenkraft aneinander vorbei gleiten.

Bei einer Trageinrichtung, wie sie hier beschrieben ist, ist die Sicherungseinrichtung insbesondere ausgebildet, um beim Verschieben des Riegels in die verriegelnde Position ein zumindest entweder taktil oder akustisch wahrnehmbares Signal zu erzeugen.

Die Sicherungseinrichtung erzeugt insbesondere ein Signal, das von einer Person, die den Riegel manuell verschiebt, taktil wahrnehmbar ist. Alternativ oder zusätzlich erzeugt die Sicherungseinrichtung ein Knacken oder ein anderes akustisch wahrnehmbares Signal, wenn der Riegel die verriegelnde Position einnimmt. Die erfindungsgemäße Trageinrichtung, wie sie hier beschrieben ist, umfasst insbesondere ferner einen Holm zum Tragen eines medizinischen Geräts oder eines medizinischen Instruments oder einer anderen Nutzlast, wobei der Holm mit dem Eingreifhaken verbindbar ist, wobei der Riegel mit dem Holm starr verbunden ist; sowie eine erste Platte, die mit dem Eingreifhaken starr verbunden ist und die eine Bohrung aufweist, wobei der Riegel in der verriegelnden Position durch die Bohrung der ersten Platte hindurchgreift, wobei in der verriegelnden Position des Riegels der Holm um eine durch den Riegel verlaufende Achse zwischen einer Loseposition und einer Festposition schwenkbar ist, wobei in der Loseposition des Holms der Riegel zwischen seiner verriegelnden Position und seiner entriegelnden Position verschiebbar ist und der Holm von dem Eingreifhaken getrennt werden kann, und wobei in der Festposition des Holms der Holm mit dem Eingreifhaken mechanisch verbunden und für eine Belastung durch eine Nutzlast vorgesehen ist.

Die Trageinrichtung umfasst zwei separate, jedoch in der Festposition des Holms miteinander verbundene Teile bzw. Einheiten. Die erste Einheit umfasst den Holm und den Riegel, die sich in zwei Richtungen erstrecken, die insbesondere parallel oder im Wesentlichen parallel zueinander sind. Die zweite Einheit umfasst den Eingreifhaken und die Bohrung. Wenn der Riegel in die Bohrung eingesetzt ist bzw. durch die Bohrung hindurchgreift, ist die erste Einheit relativ zur zweiten Einheit um eine Achse schwenkbar, die durch den Riegel und die Bohrung definiert ist, und die insbesondere parallel zum Riegel und zum Holm ist. Zur Verbindung der Trageinrichtung mit einem Geräteträger wird zunächst der Eingreifhaken in eine Öffnung am Geräteträger eingesetzt bzw. eingehängt. Danach wird der Riegel mit einer linearen Bewegung durch die Bohrung am Eingreifhaken hindurch in seine verriegelnde Position, die insbesondere in der gleichen Öffnung oder in einer benachbarten Öffnung liegt, gebracht. Am Ende dieser linearen Bewegung befindet sich die erste Einheit relativ zur zweiten Einheit in der Loseposition, in der sie durch eine entgegengesetzte lineare Bewegung wieder von der zweiten Einheit getrennt werden kann. Aus der Loseposition kann die erste Einheit in die Festposition geschwenkt werden, in der der erste Teil und der zweite Teil miteinander verbunden sind, insbesondere nicht mehr auseinander gezogen werden können.

Eine Trageinrichtung, wie sie hier beschrieben ist, umfasst insbesondere ferner eine zweite Platte, die mit dem Holm und dem Riegel starr verbunden ist, wobei in der Festposition die erste Platte und die zweite Platte formschlüssig miteinander verbunden sind.

Die formschlüssige Verbindung der beiden Platten erfolgt beispielsweise durch Schrauben oder andere pilzförmige Einrichtungen an einer der beiden Platten, die in Schlitze an der anderen Platte eingreifen.

Eine Trageinrichtung, wie sie hier beschrieben ist, umfasst insbesondere ferner eine Abdeckkappe oder eine Klammer, die über die erste Platte und die zweite Platte stülpbar ist, um die zweite Platte relativ zu der ersten Platte in der Festposition zu halten.

Die erste Platte und die zweite Platte haben insbesondere eine ähnlich oder im Wesentlichen gleiche Gestalt. Beispielsweise sind die erste Platte und die zweite Platte jeweils rechteckig mit den gleichen linearen Abmessungen. Die Trageinrichtung ist insbesondere so ausgebildet, dass in der Festposition des Holms die erste Platte und die zweite Platte im Wesentlichen deckungsgleich übereinander liegen. Die Abdeckkammer oder die Klammer liegt an gegenüberliegenden Rändern oder an allen Rändern der ersten Platte und der zweiten Platte an und hält so formschlüssig die zweite Platte relativ zu der ersten Platte und damit auch den Holm in der Festposition.

Ein Geräteträger zum Tragen eines medizinischen Geräts, eines medizinischen Instruments oder einer anderen Nutzlast umfasst ein Tragwerk und eine Öffnung an dem Tragwerk, wobei die Öffnung zum Aufnehmen eines Eingreifhakens an einer Trageinrichtung vorgesehen und ausgebildet ist.

Der Geräteträger ist insbesondere ein Gerätewagen für medizinische oder nicht-medizinische Anwendungen mit einer oder mehreren Rollen oder Rädern zum Ableiten der Gewichtskraft des Geräteträgers in einen Boden, auf dem der Geräteträger steht, wobei die Räder oder Rollen eine reibungsarme Verschiebung des Geräteträgers auf dem Boden ermöglichen. Alternativ ist der Geräteträger für eine stationäre medizinische oder nicht-medizinische Anwendung vorgesehen. Der Geräteträger ist insbesondere dazu vorgesehen und ausgebildet, ein medizinisches Gerät, ein medizinisches Instrument oder eine andere Nutzlast mittelbar mittels einer über die Öffnung mit dem Geräteträger verbundenen Trageinrichtung zu tragen. Das Tragwerk umfasst insbesondere eine oder mehrere vertikale oder im Wesentlichen vertikale Säulen oder Holme, die durch einen Boden, eine Brücke und/oder andere horizontale Einrichtungen verbunden sein können.

Bei einem Geräteträger, wie er hier beschrieben ist, ist insbesondere eine Mehrzahl von Öffnungen an dem Tragwerk vorgesehen, wobei ein Eingreifhaken an einer Trageinrichtung alternativ in eine der Mehrzahl von Öffnungen eingreifen kann.

Die Öffnungen sind insbesondere in einer Reihe übereinander und mit gleichen gegenseitigen Abständen, d. h. in einem Raster, angeordnet.

Ein Geräteträger, wie er hier beschrieben ist, umfasst insbesondere mehrere Säulen mit je einer Öffnung zum Aufnehmen eines Eingreifhakens an einer Trageinrichtung.

Bei einem Geräteträger, wie er hier beschrieben ist, umfasst das Tragwerk insbesondere mehrere Säulen, wobei an jeder der Säulen jeweils mehrere Öffnungen vorgesehen sind, wobei an jeder Säule jeweils Öffnungen mit unterschiedlichen Markierungen vorgesehen sind, und wobei Öffnungen, die im Fall einer vorgesehenen Anordnung einer Trageinrichtung gleichzeitig Hakeneinrichtungen der Trageinrichtung aufnehmen, gleiche oder entsprechende Markierungen aufweisen.

Eine vorgesehene Anordnung einer Trageinrichtung ist insbesondere eine horizontale Anordnung bzw. eine Anordnung der Trageinrichtung parallel zu einer Bodenfläche, auf der der Geräteträger steht. Die unterschiedlichen Markierungen sind insbesondere unterschiedliche farbliche Markierungen. Beispielsweise weist jede Säule mehrere Halteeinrichtungen mit jeweils einer oder mehreren Öffnungen auf, wobei benachbarte Halteeinrichtungen unterschiedliche Farben aufweisen.

Ein Geräteträger, wie er hier beschrieben ist, umfasst insbesondere ferner eine Trageinrichtung, wie sie hier beschrieben ist.

Ein Geräteträger, wie er hier beschrieben ist, umfasst insbesondere mehrere Säulen mit je einer Öffnung, wobei an der Trageinrichtung mehrere Eingreifhaken vorgesehen und dazu ausgebildet sind, gleichzeitig in je eine Öffnung einzugreifen.

Bei einem Geräteträger, wie er hier beschrieben ist, sind die Öffnungen an den Säulen und die Eingreifhaken und die Riegel an der Trageinrichtung insbesondere so ausgebildet, dass zumindest entweder ein Einführen der Eingreifhaken an der Trageinrichtung in je eine Öffnung an den Säulen oder ein Verriegeln der Verbindung zwischen einem Eingreifhaken und einer Öffnung nur möglich ist, wenn die Trageinrichtung eine von mehreren vorgesehenen Positionen einnimmt.

Vorgesehene Positionen der Trageinrichtung sind insbesondere Positionen, in denen die Trageinrichtung horizontal bzw. parallel zu einer Bodenfläche, auf der der Geräteträger steht, angeordnet ist. Der Geräteträger und die Trageinrichtung sind also so ausgebildet, dass ein schräges bzw. falsches Verbinden der Trageinrichtung mit dem Geräteträger nicht möglich ist.

Nachfolgend werden Ausführungsformen anhand der beigefügten Figuren näher erläutert. Es zeigen:
- Figur 1: eine schematische Darstellung eines medizinischen Gerätewagens;
- Figur 2: eine weitere schematische Darstellung des medizinischen Gerätewagens aus Figur 1;
- Figur 3: eine weitere schematische Darstellung des medizinischen Gerätewagens aus den Figuren 1 und 2;
- Figur 4: eine schematische Darstellung eines vertikalen Schnitts durch den medizinischen Gerätewagen aus den Figuren 1 bis 3;
- Figur 5: eine weitere schematische Darstellung des medizinischen Gerätewagens aus den Figuren 1 bis 4;
- Figur 6: eine weitere schematische Darstellung eines vertikalen Schnitts durch den medizinischen Gerätewagen aus den Figuren 1 bis 5;
- Figur 7: eine weitere schematische Darstellung eines vertikalen Schnitts durch den medizinischen Gerätewagen aus den Figuren 1 bis 6;
- Figur 8: eine weitere schematische Darstellung des medizinischen Gerätewagens aus den Figuren 1 bis 7;
- Figur 9: eine weitere schematische Darstellung eines vertikalen Schnitts durch den medizinischen Gerätewagen aus den Figuren 1 bis 8;
- Figur 10: eine schematische Darstellung einer Geräteschiene;
- Figur 11: eine schematische Darstellung eines Teils der Geräteschiene aus Figur 10;
- Figur 12: eine weitere schematische Darstellung eines Teils der Geräteschiene aus den Figuren 10 und 11;
- Figur 13: eine weitere schematische Darstellung der Geräteschiene aus den Figuren 10 bis 12;
- Figur 14: eine weitere schematische Darstellung der Geräteschiene aus den Figuren 10 bis 13;
- Figur 15: eine weitere schematische Darstellung der Geräteschiene aus den Figuren 10 bis 14;
- Figur 16: eine weitere schematische Darstellung der Geräteschiene aus den Figuren 10 bis 15.

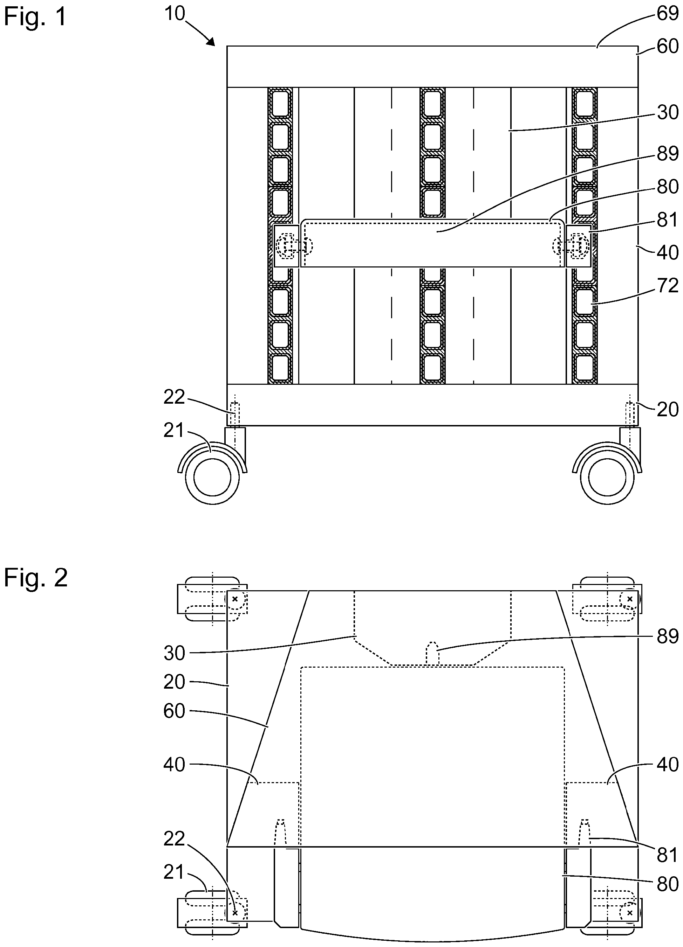

Figur 1 zeigt eine schematische Darstellung eines medizinischen Geräteträgers, insbesondere eines medizinischen Gerätewagens 10. Die Zeichenebene der Figur 1 ist vertikal bzw. bei der vorgesehenen Verwendung des medizinischen Gerätewagens 10 orthogonal zu einer horizontalen Bodenfläche, auf der der medizinische Gerätewagen 10 steht.

Der medizinische Gerätewagen 10 umfasst mehrere Flächenbauteile, nämlich einen Boden 20, eine Brücke 60 und einen Fachboden 80 als Trageinrichtung für medizinische Geräte und Instrumente, sowie mehrere Säulen 30, 40. Die Flächenbauteile 20, 60, 80 erstrecken sich im Wesentlichen parallel zur Bodenfläche, auf der der medizinische Gerätewagen 10 steht, und damit orthogonal zur Zeichenebene der Figur 1. Die Säulen 30, 40 erstrecken sich im Wesentlichen orthogonal zu den Flächenbauteilen 20, 60, 80 und parallel zur Zeichenebene der Figur 1.

Der Boden 20 ist bei der vorgesehenen Verwendung des medizinischen Gerätewagens 10 unten angeordnet. An der Unterseite des im Wesentlichen rechteckigen Bodens 20 ist an jeder Ecke des Bodens 20 eine Rolleneinheit 21 angeordnet. Jede Rolleneinheit 21 umfasst insbesondere zwei Rollen und ist um eine vertikale Schwenkachse 22 schwenkbar, so dass der medizinische Gerätewagen reibungsarm in beliebige Richtungen geschoben und rotiert werden kann. Die Brücke 60 bildet bei dem dargestellten Beispiel den oberen Abschluss des medizinischen Gerätewagens 10.

Der medizinische Gerätewagen 10 weist eine Vorderseite auf, die dafür vorgesehen ist, medizinischem Personal zugewandt zu sein, und die bei der Darstellung in Figur 1 dem Betrachter zugewandt ist. Die zur Vorderseite entgegengesetzte Rückseite des medizinischen Gerätewagens ist bei der Darstellung in Figur 1 vom Betrachter abgewandt. Von der Vorderseite aus kann der Fachboden 80 in den medizinischen Gerätewagen 10 eingesetzt werden, und von der Vorderseite aus sind auf oder in dem medizinischen Geräteträger 10 abgestellte oder gelagerte oder bereitgehaltene medizinische Geräte oder Instrumente zugänglich.

Eine erste Säule 30 ist an der Rückseite des medizinischen Gerätewagens 10 mittig angeordnet. Die zweiten Säulen 40 sind symmetrisch zueinander an den Seiten des medizinischen Gerätewagens 10 nahe der Vorderseite des medizinischen Gerätewagens 10 angeordnet. Jede Säule 30, 40 weist eine Mehrzahl von Öffnungen 72 auf. Die Öffnungen 72 sind an jeder Säule 30, 40 in einem regelmäßigen Raster angeordnet und jeweils in Richtung zur Vorderseite des medizinischen Gerätewagens 10 hin offen.

Der Fachboden 80 weist einen oder mehrere Stifte 89 und zwei oder mehr Eingreifhaken 81 auf, die den Öffnungen 72 zugewandt und zum Eingreifen in je eine Öffnung 72 vorgesehen und ausgebildet sind. Entsprechend sind der oder die Stifte 89 und die Eingreifhaken 81 bei der Darstellung in Figur 1 vom Betrachter abgewandt und von anderen Teilen des Fachbodens 80 verdeckt und deshalb in Figur 1 lediglich in gestrichelter Kontur angedeutet. Der oder die Stifte 89 sind mittig oder nahe der Mitte an der hinteren Kante des Fachbodens 80 angeordnet, um in je eine Öffnung 72 in der ersten Säule 30 einzugreifen. Abweichend von der Darstellung in Figur 1 kann die erste Säule 30 zwei oder mehr vertikale Reihen von Öffnungen 72 aufweisen. Die Eingreifhaken 81 sind vorgesehen, angeordnet und ausgebildet, um in je eine Öffnung 72 in den seitlich angeordneten zweiten Säulen 40 einzugreifen.

Der Fachboden 80 kann in verschiedenen Höhen bzw. in verschiedenen Abständen vom Boden 20 und von der Brücke 60 angeordnet werden. An einem oder an beiden Eingreifhaken 81 und/oder am Stift 89 sind in Figur 1 nicht dargestellte Einrichtungen zum Verriegeln des Fachbodens 80 an den Säulen 30, 40 vorgesehen, die in Figur 1 nicht dargestellt sind. Insbesondere werden ein oder beide Eingreifhaken 81 und/oder der Stift 89 jeweils in einer Öffnung 72 verriegelt, so dass ein unbeabsichtigtes Lösen des Fachbodens 80 aus seiner vorgesehenen Position und ein nachfolgendes Herabfallen verhindert werden.

Der medizinische Gerätewagen 10 kann modifiziert werden, indem der Fachboden 80 durch ein oder mehrere Module mit Auszügen und/oder offenen oder durch Klappen oder Türen verschließbaren Fächern und/oder durch weitere Fachböden 80 ersetzt oder ergänzt wird. Bei der dargestellten Konfiguration können insbesondere auf dem Boden 20 und auf dem Fachboden 80 und optional zusätzlich auf der Brücke 60 medizinische Geräte, medizinische Instrumente und/oder andere Nutzlast angeordnet werden.

Figur 2 zeigt eine weitere schematische Darstellung des medizinischen Gerätewagens 10 aus Figur 1. Figur 2 zeigt eine Draufsicht, d.h. die Zeichenebene der Figur 2 ist horizontal bzw. parallel zu einer Bodenfläche, auf der der medizinische Gerätewagen 10 in seiner vorgesehenen Anordnung steht, und damit orthogonal zur Zeichenebene der Figur 1. Die bei der Darstellung in Figur 1 vom Betrachter abgewandte Rückseite des medizinischen Gerätewagens 10 liegt in Figur 2 oben, die bei der Darstellung in Figur 1 dem Betrachter zugewandte Vorderseite des medizinischen Gerätewagens 10 liegt bei der Darstellung in Figur 2 unten.

In Figur 2 ist die rechteckige Grundform des Bodens 20 mit je einer Rolleneinheit 21 an jeder Ecke erkennbar. Die vertikalen Schwenkachsen 22 der Rolleneinheiten 21 sind orthogonal zur Zeichenebene der Figur 2.

Über dem Boden 20 erstrecken sich die Säulen 30, 40 im Wesentlichen orthogonal zur Zeichenebene der Figur 2. Jede Säule 30, 40 weist einen im Wesentlichen polygonalen Querschnitt auf. Die erste Säule 30 ist mittig an der Rückseite des medizinischen Gerätewagens angeordnet. Die zweiten Säulen 40 sind symmetrisch zueinander ausgebildet und symmetrisch zueinander an den Seiten des medizinischen Gerätewagens 10 angeordnet. Bei dem dargestellten Beispiel sind die zweiten Säulen 40 näher an der Vorderseite als an der Rückseite des medizinischen Gerätewagens 10 angeordnet. Die Säulen 30, 40 sind bei der Blickrichtung der Figur 2 durch die Brücke 60 verdeckt. Deshalb sind in Figur 2 lediglich die äußeren Konturen der Querschnitte der Säulen 30, 40 in gestrichelten Linien angedeutet.

Die Brücke 60 weist bei dem dargestellten Beispiel eine im Wesentlichen trapezförmige Grundform auf und verdeckt bei der Blickrichtung der Figur 2 einen Teil des Fachbodens 80, der deshalb lediglich in gestrichelten Linien angedeutet ist.

Der Fachboden 80 weist an beiden Seiten symmetrisch zueinander angeordnete Eingreifhaken 81 auf, die in korrespondierende Öffnungen 72 (vgl. Figur 1) in den zweiten Säulen 40 eingreifen. Ferner weist der Fachboden 80 einen Stift 89 auf, der in eine korrespondierende Öffnung 72 (vgl. Figur 1) in der ersten Säule 30 eingreift.

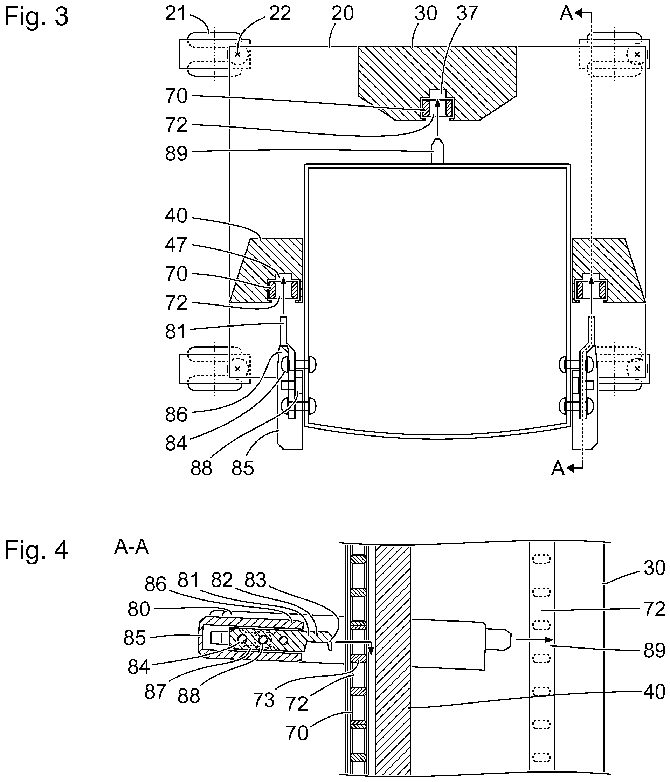

Figur 3 zeigt eine weitere schematische Darstellung des medizinischen Gerätwagens 10 aus den Figuren 1 und 2. Die Orientierung der Zeichenebene der Figur 3 entspricht derjenigen der Figur 2. Im Unterschied zur Figur 2 sind in Figur 3 die Brücke 60 nicht und die Säulen 30, 40 im Schnitt entlang einer horizontalen Ebene dargestellt. Ferner sind Teile des Fachbodens 80 transparent dargestellt, um ihren innere Aufbau zu zeigen.

Die Säulen 30, 40 weisen jeweils zur Vorderseite des medizinischen Gerätewagens 10 hin offene Kanäle 37, 47 auf, in denen Rasterleisten 70 angeordnet sind. Die Kanäle 37, 47 sind so ausgebildet, dass die Rasterleisten 70 formschlüssig in den Kanälen 37, 47 gehalten sind. Alternativ oder zusätzlich können die Rasterleisten 70 durch Schrauben, Niete oder auf andere Weise formschlüssig, stoffschlüssig oder reibschlüssig in den Kanälen 37, 47 in den Säulen 30, 40 gehalten sein. Ferner können die Öffnungen 72 abweichend von der Darstellung in Figur 3 unmittelbar in den Säulen 30, 40 ausgebildet sein, beispielsweise in Gestalt von Reihen von Bohrungen.

In jedem Kanal 37, 47 an einer Säule 30, 40 sind mehrere Rasterleisten 70 eingesetzt, wobei jede Rasterleiste 70 eine oder mehrere Öffnungen 72 aufweist. Die Rasterleisten 70 im Kanal 37, 47 einer Säule 30, 40 weisen unterschiedliche Markierungen, insbesondere unterschiedliche Farben auf. Dies ist in Figur 1 durch unterschiedliche Schraffuren (nämlich in einem Fall von links oben nach rechts unten und im anderen Fall von links unten nach rechts oben) angedeutet. Die Markierungen sind so gewählt, dass korrespondierende Öffnungen 72 an unterschiedlichen Säulen 30, 40, d.h. Öffnungen 72, in die bei vorgesehener, insbesondere horizontaler Anordnung des Fachbodens 80 gleichzeitig dessen Eingreifhaken 81 und dessen Stift 89 eingreifen, gleich markiert sind. Die Markierungen vereinfachen beim Einsetzen des Fachbodens 80 das richtige Einführen der Eingreifhaken 81 und des Stifts 89 in die Öffnungen 72 und vermindern das Risiko einer falschen, insbesondere schrägen Positionierung des Fachbodens 80.

Die Querschnitte der Säulen 30, 40 sind in Figur 3 stark vereinfacht homogen dargestellt. Abweichend von der Darstellung in Figur 3 sind die Säulen 30, 40 aus Profilteilen aus Metall und/oder Kunststoff, insbesondere aus Strangpressprofilen gebildet oder zusammengesetzt. Im Inneren der Säulen 30, 40 können Hohlräume bzw. Kanäle zur Anordnung von Fluidleitungen und Leitungen zur elektrischen oder optischen Übertragung von Leistung und/oder Daten vorgesehen sein.

Der Fachboden 80 ist in Figur 3 in einer Position dargestellt, in der er noch nicht mit den Säulen 30, 40 des medizinischen Gerätewagens 10 verbunden ist. Insbesondere greifen die Eingreifhaken 81 an den Seiten des Fachbodens 80 und der Stift 89 am rückseitigen Rand des Fachbodens 80 noch nicht in die Öffnungen 72 ein. Durch Pfeile ist eine Bewegung des Fachbodens 80, insbesondere der Eingreifhaken 81 und des Stifts 89 in die Öffnungen 72 hinein, angedeutet, mit der der Fachboden 80 mit den Säulen 30, 40 und damit mit dem medizinischen Gerätewagen 10 mechanisch verbunden werden kann.

Die Eingreifhaken 81 sind beispielsweise durch Blechstreifen, beim dargestellten Beispiel durch gekröpften Blechstreifen gebildet. Jeder Eingreifhaken 81 ist mittels zweier Niete 84 mit dem Fachboden 80 starr verbunden. Zwischen den Nieten und zwischen dem Eingreifhaken 81 und dem Fachboden 80 sind jeweils ein oder mehrere Schraubenköpfe 88 von Schrauben angeordnet, die entweder in den Eingreifhaken 81 oder in den Rand des Fachbodens 80 geschraubt sind. Die Funktion dieses Schraubenkopfs 88 ist anhand der Figuren 4, 6, 8 und vor allem 9 beschrieben.

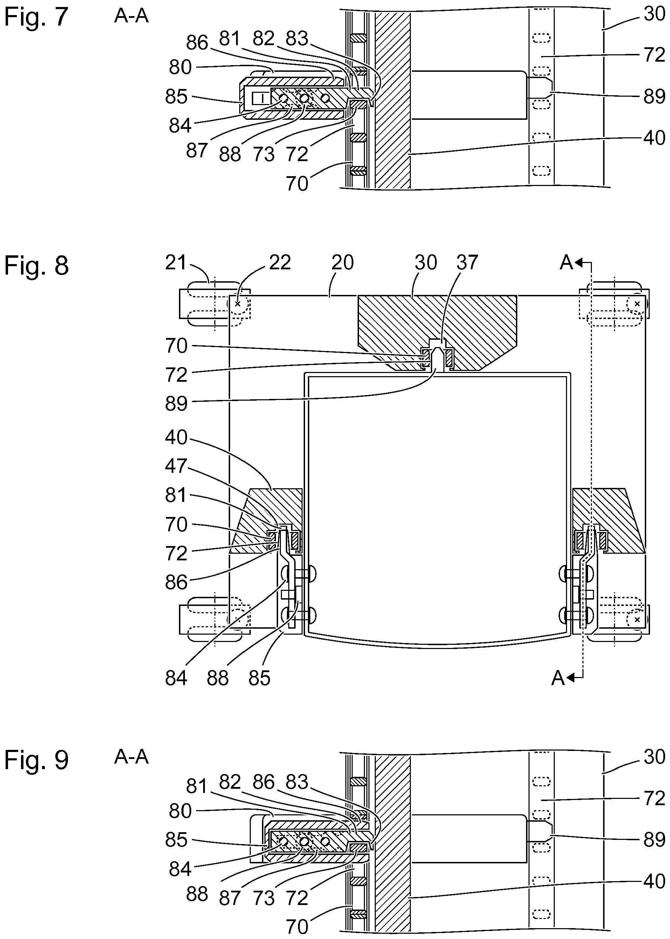

An jedem Eingreifhaken 81 ist ein Schieber 85 mit zwei Riegeln 86 angeordnet. Der Schieber 85 ist transparent dargestellt bzw. nur durch seine äußeren Konturen angedeutet, um durch ihn verdeckte Merkmale sichtbar zu machen. Jeder Schieber 85 ist zwischen einer in Figur 3 gezeigten entriegelnden Position und einer verriegelnden Position verschiebbar. Die Wirkung des Riegels 86 am Schieber 85 ist insbesondere anhand der Figur 9 beschrieben.

Figur 4 zeigt eine schematische Darstellung eines Schnitts entlang der in Figur 3 angedeuteten Fläche A-A. Die Fläche A-A ist vertikal und damit orthogonal zur Zeichenebene der Figur 3.

Der Eingreifhaken 81 weist einen geraden Abschnitt 82 und einen gegenüber dem geraden Abschnitt 82 abgewinkelten Abschnitt 83 auf. Zwischen den Nieten 84 ist die Schraube mit dem Schraubenkopf 88 angeordnet. Die Kontur des Schraubenkopfs 88 und Ränder des Schiebers 85, die durch das den Eingreifhaken 81 bildende Bauteil verdeckt sind, sind durch gestrichelte Linien angedeutet. Der Schieber 85 weist, soweit er nicht die Riegel 86 bildet, einen C-förmigen Querschnitt auf, der das den Eingreifhaken 81 bildende Bauteil hintergreift.

Die gestrichelt angedeuteten Ränder des Schiebers 85 bilden mehrere Rastnasen 87, die mit dem Schraubenkopf 88 wechselwirken. Durch die Wechselwirkung zwischen den Rastnasen 87 am Schieber 85 und dem Schraubenkopf 88 werden der Schieber 85 und vor allem die Riegel 86 in der in den Figuren 3 und 4 gezeigten entriegelnden Position und in der anhand der Figur 9 dargestellten verriegelnden Position gehalten.

Ein abgewinkelter Pfeil deutet an, wie der Eingreifhaken 81 in eine Öffnung 72 in einer Rasterleiste 70 in der Säule 40 eingeführt werden kann. Ein weiterer Pfeil deutet an, wie gleichzeitig der Stift 89 an der rückseitigen (in Figur 4 rechten) Kante des Fachbodens 80 in eine Öffnung 72 in der Säule 30 eingeführt werden kann.

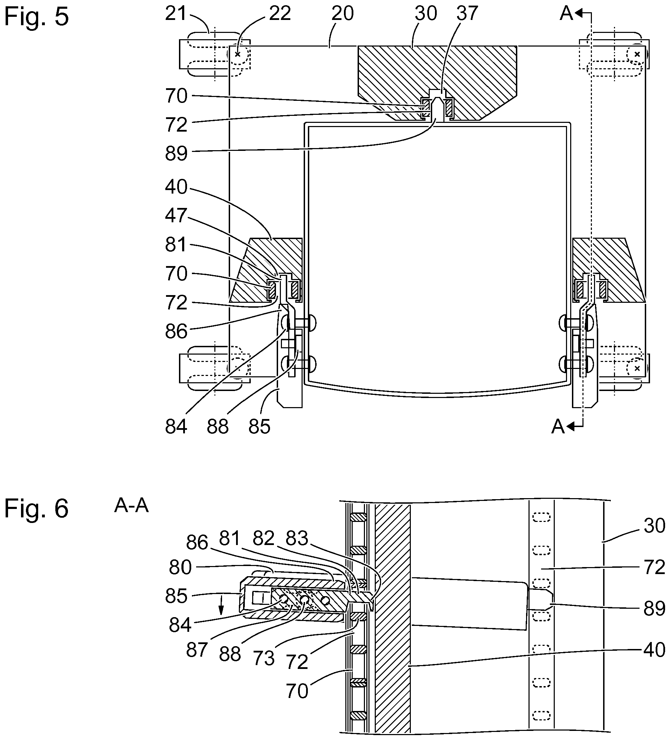

Figur 5 zeigt eine weitere schematische Darstellung des medizinischen Gerätewagens 10 aus den Figuren 1 bis 4. Die Art der Darstellung und insbesondere die Orientierung der Zeichenebene entsprechen denjenigen der Figur 3.

In Figur 5 ist der Fachboden 80 in einer Position gezeigt, in der die Eingreifhaken 81 und der Stift 89 bereits in drei korrespondierende Öffnungen in Rasterleisten 70 in den Säulen 30, 40 eingeführt ist.

Figur 6 zeigt eine weitere schematische Darstellung des medizinischen Gerätewagens aus den Figuren 1 bis 5. Die Art der Darstellung entspricht derjenigen der Figur 4. Die dargestellte Situation entspricht derjenigen der Figur 5. Der Eingreifhaken 81 greift vollständig in eine Öffnung 72 in einer Rasterleiste 70 in einer zweiten Säule 40 ein, wobei er die Rasterleiste 70 vollständig durchgreift. Der Stift 89 greift ebenfalls in eine korrespondierende Öffnung 72 in der ersten Säule 30 ein.

Figur 7 zeigt eine weitere schematische Darstellung des medizinischen Gerätewagens aus den Figuren 1 bis 6. Die Art der Darstellung entspricht derjenigen der Figuren 4 und 6. Gegenüber der in Figur 6 gezeigten Situation ist der Fachboden 80 entsprechend dem Pfeil in Figur 6 abgesenkt, so dass der gerade Abschnitt 82 des Eingreifhakens 81 auf einem Steg 73 zwischen zwei Öffnungen 72 in der Rasterleiste 70 in der zweiten Säule 40 aufliegt und der gegenüber dem geraden Abschnitt 82 abgewinkelte Abschnitt 83 des Eingreifhakens 81 den Steg 73 hintergreift. Dadurch ist ein Trennen des Fachbodens 80 von den Säulen 30, 40 durch eine rein horizontale Bewegung formschlüssig unterbunden.

Figur 8 zeigt eine weitere schematische Darstellung des medizinischen Gerätewagens aus den Figuren 1 bis 7. Die Art der Darstellung entspricht derjenigen der Figuren 3 und 5. Die in Figur 8 gezeigte Situation unterscheidet sich von der in Figur 7 gezeigten dadurch, dass die Schieber 85 mit den Riegeln 86 in eine verriegelnde Position geschoben sind, in der die Riegel 86 in Öffnungen 72 in den Säulen 40 eingreifen.

Figur 9 zeigt eine weitere schematische Darstellung des medizinischen Gerätewagens aus den Figuren 1 bis 8. Die Art der Darstellung entspricht derjenigen der Figuren 4, 6 und 7. Die dargestellte Situation entspricht der in Figur 8 dargestellten Situation. Einer der beiden Riegel 86 am Schieber 85 greift in die gleiche Öffnung 72 ein, in die der Eingreifhaken 81 eingreift. Der zweite Riegel 86 greift in eine benachbarte Öffnung 72 ein. Jeder einzelne Riegel 86 unterbindet bereits für sich genommen eine Bewegung des Schiebers 85 und damit auch eine Bewegung des Eingreifhakens 81 in vertikaler Richtung. Dadurch verriegeln die Riegel 86 den Eingreifhaken 81 in der in Figur 9 gezeigten Position, in der der gegenüber dem geraden Abschnitt 82 abgewinkelte Abschnitt 83 des Eingreifhakens 81 den Steg 73 hintergreift. Der Fachboden 80 kann nicht mehr von den Säulen 30, 40 gelöst werden. Der Fachboden 80 kann erst dann wieder von den Säulen 30, 40 gelöst werden, wenn der Schieber 85 mit den Riegeln 86 zurück in die in den Figuren 3 bis 7 gezeigte entriegelnde Position geschoben ist.

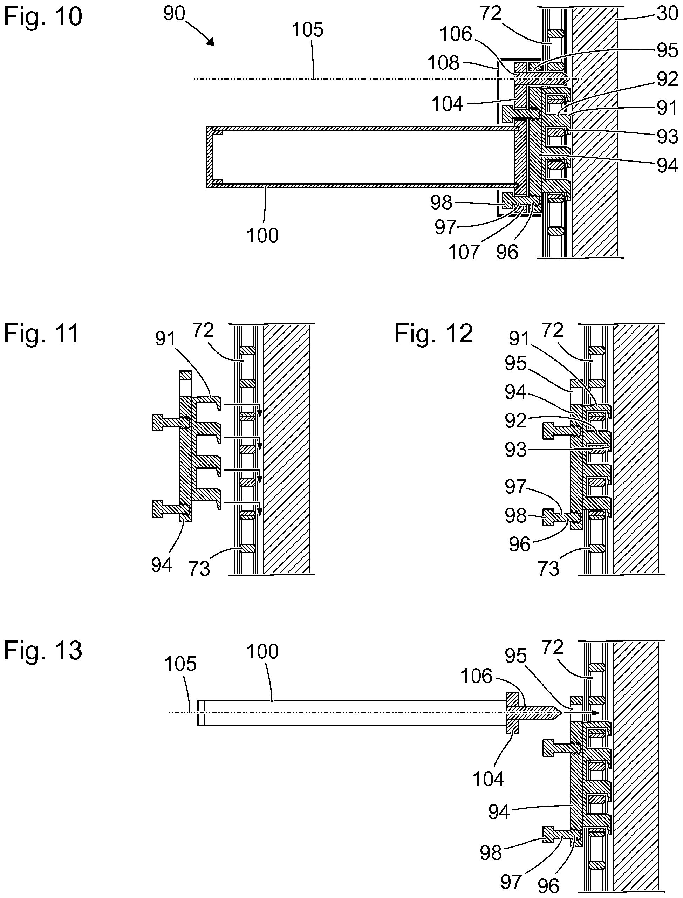

Figur 10 zeigt eine schematische Darstellung der erfindungsgemäßen Trageinrichtung, nämlich einer Geräteschiene 90 an einer Säule 30 des medizinischen Gerätewagens aus den Figuren 1 bis 9. Die Geräteschiene 90 kann alternativ zu oder zusätzlich zu dem anhand der Figuren 3 bis 9 dargestellten Fachboden mit dem medizinischen Gerätewagen 10 verbunden werden. Die Geräteschiene 90 umfasst einen Holm 100, der bei der vorgesehenen Verwendung der Geräteschiene 90 insbesondere horizontal oder im Wesentlichen horizontal und damit orthogonal zur Säule 30 angeordnet ist. Die Geräteschiene 90 umfasst mehrere Eingreifhaken 91 mit jeweils einem geraden Abschnitt 92 und einem gegenüber dem geraden Abschnitt 92 abgewinkelten Abschnitt 93. Die Eingreifhaken 91 ähneln oder entsprechen insbesondere den Eingreifhaken des anhand der Figuren 3 bis 9 dargestellten Fachbodens. Die Eingreifhaken 91 sind mit einer ersten Platte 94 starr verbunden, beispielsweise durch Schweißen oder Löten gefügt. In der ersten Platte 94 ist eine Bohrung 95 vorgesehen. An der von den Eingreifhaken 91 abgewandten Seite der ersten Platte 94 sind zwei Schrauben 96 vorgesehen. Die Schrauben 96 sind so in die erste Platte 94 eingesetzt, dass nicht nur ihre Köpfe 98, sondern auch jeweils ein Teil ihres Schafts 97 über die erste Platte 94 übersteht.

Die Geräteschiene 90 umfasst ferner eine zweite Platte 104, mit der der Holm 100 starr verbunden ist. Die mit der ersten Platte 94 verbundenen Schrauben 96 durchgreifen Schlitze 107 in der zweiten Platte 104, wobei die Schäfte 97 der Schrauben 96 in den Schlitzen 107 liegen und die Köpfe 98 der Schrauben 96 einerseits und die erste Platte 94 andererseits an entgegengesetzten Seiten der zweiten Platte 104 anliegen. Ein Stift 106 ist mit der zweiten Platte 104 starr verbunden, insbesondere in eine korrespondierende Bohrung in der zweiten Platte 104 eingesetzt und mit dieser reibschlüssig, stoffschlüssig und/oder formschlüssig verbunden. Der Stift 106 steht gegenüber der zweiten Platte 104 in einer zum Holm 100 entgegengesetzten Richtung über. Der Stift 106 ist zu einer horizontalen Achse 105 rotationssymmetrisch.

Der Stift 106 greift durch die Bohrung 95 in der ersten Platte 94 hindurch und in eine Öffnung 72 an der Säule 30 ein, in die insbesondere gleichzeitig einer der Eingreifhaken 91 eingreift. Der Stift 106 wirkt als Riegel, der in seiner in Figur 10 gezeigten verriegelnden Position ein Anheben der Eingreifhaken 91 und damit ein Trennen der Geräteschiene 90 von der Säule 30 formschlüssig unterbindet. Eine Abdeckkappe 108 umschließt die Platten 94, 104 und sichert deren nachfolgend beschriebene Verbindung.

Nachfolgend ist anhand der Figuren 11 bis 16 beschrieben, wie die Geräteschiene 90 mit der Säule 30 verbunden werden kann.

Figur 11 zeigt eine schematische Schnittdarstellung der Säule 30 und der ersten Platte 94 mit den Eingreifhaken 91 und den Schrauben 96. Die Art der Darstellung, insbesondere die Schnittebene, entspricht derjenigen der Figur 10.

In Figur 11 ist eine Situation gezeigt, in der die erste Platte 94 und die Eingreifhaken 91 von der Säule 30 beabstandet sind. Durch Pfeile ist eine Bewegung angedeutet, mit der die Eingreifhaken 91 in Öffnungen 72 in Rasterleisten 70 in der Säule 30 eingesetzt werden können.

Figur 12 zeigt eine weitere schematische Schnittdarstellung der Säule 30 und der ersten Platte 94 mit den Eingreifhaken 91. Die Art der Darstellung, insbesondere die Schnittebene, entspricht denjenigen der Figuren 10 und 11.

In Figur 12 sind die Haken 91 entsprechend den Pfeilen in Figur 11 in Öffnungen 72 eingesetzt. Dazu wurden zunächst mit einer horizontalen Bewegung parallel zu den geraden Abschnitten 92 der Eingreifhaken 91 die Eingreifhaken 91 vollständig in die Öffnungen 72 eingeführt und dann mit einer vertikalen Bewegung die gegenüber den geraden Abschnitten 92 abgewinkelten Abschnitte 93 hinter die Stege 73 zwischen den Öffnungen 72 bewegt, so dass die abgewinkelten Abschnitte 93 die Stege 73 hintergreifen.

Figur 13 zeigt eine weitere schematische Schnittdarstellung der Geräteschiene 90 aus den Figuren 10 bis 12. Die Art der Darstellung, insbesondere die Schnittebene, entspricht derjenigen der Figuren 10 bis 12.

Durch einen Pfeil ist angedeutet, wie mit einer horizontalen Bewegung der Einheit aus dem Holm 100, der zweiten Platte 104 und dem Stift 106 der Stift 106 durch die Öffnung 95 in der ersten Platte 94 hindurch und in eine Öffnung 72 in der Rasterleiste 70 eingeführt werden kann. Dabei ist die in sich starre Einheit aus Holm 100, zweiter Platte 104 und Stift 106 um die Achse 105 um einen Winkel von ca. 90 Grad gegenüber der in Figur 10 gezeigten Position gedreht.

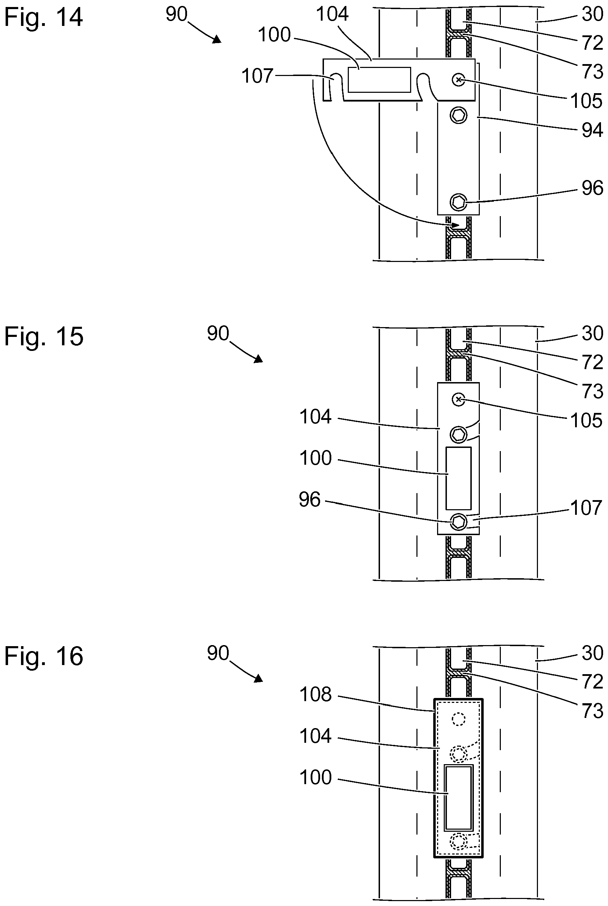

Figur 14 zeigt eine weitere schematische Darstellung der Geräteschiene 90 aus den Figuren 10 bis 13. Die Art der Darstellung entspricht derjenigen der Figur 1, die dargestellte Situation entspricht der in Figur 13 gezeigten.

Die zweite Platte 104 weist zwei Schlitze 107 zum Aufnehmen der Schäfte 97 (vgl. Figuren 10 bis 13) der Schrauben 96 auf. Nach dem vollständigen Einführen des Stifts 106durch die Bohrung 95 in der ersten Platte 94 hindurch in eine Öffnung 72, d.h. wenn die zweite Platte 104 an der ersten Platte 94 anliegt, kann die Einheit aus Holm 100, zweiter Platte 104 und Stift 106 um die durch den Stift 106 und die Bohrung 95 (vgl. Figuren 10 bis 13) in der ersten Platte 94 definierte Achse 105 geschwenkt werden. Diese Schwenkbewegung ist in Figur 14 durch einen Pfeil angedeutet.

Figur 15 zeigt eine weitere schematische Darstellung der Geräteschiene aus den Figuren 10 bis 14. Die Art der Darstellung, insbesondere die Orientierung der Zeichenebene, entspricht derjenigen der Figur 14. Figur 15 zeigt die nach dem Schwenken der Einheit aus Holm 100, zweiter Platte 104 und Stift 106 entsprechend dem in Figur 14 gezeigten Pfeil die auch in Figur 10 gezeigte Festposition. In dieser Festposition durchgreifen die Schäfte 97 (vgl. Figuren 10 bis 13) der Schrauben 96 die Schlitze 107 in der zweiten Platte 104, und die Köpfe 98 der Schrauben 96 halten die zweite Platte 104 formschlüssig an der ersten Platte 94. Der Formschluss zwischen den Schrauben 96 an der ersten Platte 94 einerseits und den Schlitzen 107 der zweiten Platte 104 andererseits ermöglicht nicht nur eine Übertragung von Kräften und Momenten vom Holm 100 zu den Eingreifhaken 91, sondern hält ferner auch den Stift 106 in seiner die Verbindung zwischen Eingreifhaken 91 und Öffnungen 72 verriegelnden Position.

Figur 16 zeigt eine weitere schematische Darstellung der Geräteschiene 90 aus den Figuren 10 bis 15. Die Art der Darstellung, insbesondere die Orientierung der Zeichenebene, entspricht derjenigen der Figuren 14 und 15. In Figur 16 ist die Abdeckkappe 108 wie auch in Figur 10 gezeigt, über die Platten 94, 104 gestülpt. Die Abdeckkappe 108 kann in der in den Figuren 10 und 16 gezeigten Position durch in den Figuren nicht gezeigte Rastnasen oder durch andere Einrichtungen gehalten werden. Die Abdeckkappe 108 kann ausgebildet sein, um beim Erreichen der in den Figuren 10 und 16 gezeigten Position ein für Menschen taktil und/oder akustisch wahrnehmbares Signal zu erzeugen. Die Abdeckkappe 108 hält die zweite Platte 104 in der in den Figuren 10, 15 und 16 gezeigten Position relativ zur ersten Platte 94 und sichert damit die formschlüssige Verbindung zwischen den beiden Einheiten der Geräteschiene 90 und die Verriegelung der Verbindung zwischen der Geräteschiene 90 und der Säule 30.

### Bezugszeichen

- 10: Medizinischer Geräteträger

- 20: Boden des medizinischen Geräteträger 10
- 21: Rolleneinheit am Boden 20
- 22: vertikale Schwenkachse der Rolleneinheit 21 (Lenkbarkeit)

- 30: erste Säule des medizinischen Geräteträgers 10 (hinten Mitte)
- 37: Kanal an der ersten Säule 30 für Rasterleisten 70

- 40: zweite Säule des medizinischen Geräteträger 10 (vorn links, rechts, baugleich spiegelsymmetrisch angeordnet)
- 47: Kanal an der zweiten Säule 40 für Rasterleisten 70

- 60: Brücke

- 70: Rasterleiste an einer ersten Säule 30 oder an der zweiten Säule 40
- 72: Öffnung in der Rasterleiste 70
- 73: Steg zwischen zwei Öffnungen 72 in der Rasterleiste 70

- 80: Fachboden (Trageinrichtung)
- 81: Eingreifhaken an Fachboden 80
- 82: gerader Abschnitt am Eingreifhaken 81
- 83: abgewinkelter Abschnitt am Eingreifhaken 81
- 84: Niet zur Befestigung des Eingreifhakens 81 an der Trageinrichtung 80
- 85: Schieber am Fachboden 80
- 86: Riegel am Schieber 85 zum Verriegeln der Verbindung zwischen Eingreifhaken 81 und Öffnung 72
- 87: Rastnase am Schieber 85
- 88: Schraubenkopf am Eingreifhaken als Gegenstück zu Rastnase 87 am Schieber 85
- 89: Stift am Fachboden 80 zum Eingreifen in Öffnung 72

- 90: Geräteschiene (Trageinrichtung)
- 91: Eingreifhaken an Geräteschiene 90
- 92: gerader Abschnitt am Eingreifhaken 91
- 93: abgewinkelter Abschnitt am Eingreifhaken 91
- 94: erste Platte an Eingreifhaken 91
- 95: Bohrung in erster Platte 94
- 96: Schraube an erster Platte 94 zum Eingreifen in Schlitz 107 an zweiter Platte 104
- 97: Schaft der Schraube 96
- 98: Kopf der Schraube 96
- 100: Holm
- 104: zweite Platte an einem Ende des Holms 100
- 105: Achse, um die der Holm 100 und die zweite Platte 104 schwenkbar sind
- 106: Stift (Riegel) an der zweiten Platte 104 zum Verriegeln der Verbindung zwischen Eingreifhaken 91 und Öffnung 72
- 107: Schlitz in zweiter Platte 104 zum Aufnehmen der Schraube 96 an erster Platte 94
- 108: Abdeckkappe zum Halten des Holms 100 und der zweiten Platte 104 in der Festposition

## Patentansprüche

1. Trageinrichtung (90) zur lösbaren Verbindung mit einem Geräteträger (10) und zum Tragen eines medizinischen Geräts, eines medizinischen Instruments oder einer anderen Nutzlast in einem medizinischem Behandlungsraum, mit: einem Eingreifhaken (91) zum Eingreifen in eine Öffnung (72) an einem Geräteträger (10); einem Riegel (106) zum Verriegeln einer Verbindung zwischen dem Eingreifhaken (91) und einer Öffnung (72), in die der Eingreifhaken (91) eingreift, **gekennzeichnet durch** einen Holm (100) zum Tragen eines medizinischen Geräts oder eines medizinischen Instruments oder einer anderen Nutzlast, wobei der Holm (100) mit dem Eingreifhaken (91) verbindbar ist, wobei der Riegel (106) mit dem Holm (100) starr verbunden ist; eine erste Platte (94), die mit dem Eingreifhaken (91) starr verbunden ist und die eine Bohrung (95) aufweist, wobei der Riegel (106) in der verriegelnden Position durch die **Bohrung (95)** der **ersten Platte (94) hindurchgreift**, wobei in der verriegelnden Position des Riegels (106) der Holm (100) um eine durch den Riegel (106) verlaufende Achse (105) zwischen einer Loseposition und einer Festposition schwenkbar ist, wobei in der Loseposition des Holms (100) der Riegel (106) zwischen seiner verriegelnden Position und seiner entriegelnden Position verschiebbar ist und der Holm (100) von dem Eingreifhaken (91) getrennt werden kann, wobei in der Festposition des Holms (100) der Holm (100) mit dem Eingreifhaken (91) mechanisch verbunden und für eine Belastung durch eine Nutzlast vorgesehen ist.

2. Trageinrichtung (90) nach dem vorangehenden Anspruch, bei der der Eingreifhaken (91) einen geraden Abschnitt (92) zum Eingreifen in eine Öffnung (72) und einen gegenüber dem geraden Abschnitt (92) abgewinkelten Abschnitt (93) zum Hintergreifen eines Rands (73) der Öffnung (72) umfasst, der Riegel (106) parallel zu dem geraden Abschnitt (92) des Eingreifhakens (91) zwischen einer entriegelnden Position und einer verriegelnden Position verschiebbar ist.

3. Trageinrichtung (90) nach dem vorangehenden Anspruch, bei der der Riegel (106) vorgesehen und ausgebildet ist, um in der entriegelnden Position nicht neben dem geraden Abschnitt (92) des Eingreifhakens (91) angeordnet zu sein, in der verriegelnden Position neben dem geraden Abschnitt (92) des Eingreifhakens (91) angeordnet zu sein.

4. Trageinrichtung (90) nach einem der vorangehenden Ansprüche, bei der der Riegel (106) vorgesehen und ausgebildet ist, um in der entriegelnden Position nicht in eine Öffnung (72) an einem Geräteträger (10), in die der Eingreifhaken (91) eingreift, einzugreifen, in der verriegelnden Position in eine Öffnung (72) an einem Geräteträger (10), in die der Eingreifhaken (91) eingreift, einzugreifen.

5. Trageinrichtung (90) nach Anspruch 1, ferner mit: einer zweiten Platte (104), die mit dem Holm (100) und dem Riegel (106) starr verbunden ist, wobei in der Festposition die erste Platte (94) und die zweite Platte (104) formschlüssig miteinander verbunden sind.

6. Trageinrichtung (90) nach dem vorangehenden Anspruch, ferner mit: einer Abdeckkappe (108) oder eine Klammer, die über die erste Platte (94) und die zweite Platte (104) stülpbar ist, um die zweite Platte (104) relativ zu der ersten Platte (94) in der Festposition zu halten.

7. Geräteträger (10) zum Tragen eines medizinischen Geräts, eines medizinischen Instruments oder einer anderen Nutzlast, **gekennzeichnet durch** ein Tragwerk (20, 30, 40, 60); einer Öffnung (72) an dem Tragwerk (30, 40), wobei die Öffnung (72) zum Aufnehmen eines Eingreifhakens (91) an einer Trageinrichtung (90) vorgesehen und ausgebildet ist und einer Trageinrichtung (90) nach einem der Ansprüche 1 bis 6 ist.

8. Geräteträger (10) nach dem vorangehenden Anspruch, bei dem eine Mehrzahl von Öffnungen (72) an dem Tragwerk (30, 40) vorgesehen ist, wobei ein Eingreifhaken (91) an einer Trageinrichtung (90) alternativ in eine der Mehrzahl von Öffnungen (72) eingreifen kann.

9. Geräteträger (10) nach dem vorangehenden Anspruch bei dem das Tragwerk mehrere Säulen (30, 40) umfasst, an jeder Säule (30, 40) jeweils mehrere Öffnungen (72) vorgesehen sind, an jeder Säule (30, 40) jeweils Öffnungen (72) mit unterschiedlichen Markierungen vorgesehen sind Öffnungen (72), die im Fall einer vorgesehenen Anordnung einer Trageinrichtung (90) gleichzeitig Hakeneinrichtungen (91) der Trageinrichtung (90) aufnehmen, gleiche oder entsprechende Markierungen aufweisen.

## Claims

1. Support device (90) for releasable connection to an equipment carrier (10), and for supporting a medical appliance, a medical instrument or another payload in a medical treatment room, comprising an engagement hook (91) for engaging in an opening (72) on an equipment carrier (10) and a bolt (106) for locking a connection between the engagement hook (91) and an opening (72) in which the engagement hook (91) engages, **characterized by** a spar (100) for supporting a medical appliance or a medical instrument or another payload, wherein the spar (100) is connectable to the engagement hook (91), wherein the bolt (106) is rigidly connected to the spar (100); a first plate (94), which is rigidly connected to the engagement hook (91) and which has a bore (95), wherein the bolt (106), in the locking position, engages through the bore (95) of the first plate (94), wherein, in the locking position of the bolt (106), the spar (100) is pivotable between a loose position and a stable position about an axis (105) extending through the bolt (106), wherein, in the loose position of the spar (100), the bolt (106) is movable between its locking position and its unlocking position and the spar (100) can be separated from the engagement hook (91), wherein, in the stable position of the spar (100), the spar (100) is mechanically connected to the engagement hook (91) and is loaded with a payload.

2. Support device (90) according to the preceding claim, in which the engagement hook (91) comprises a straight portion (92) for engaging in an opening (72), and a portion (93) which is angled in relation to the straight portion (92) and is for engaging behind an edge (73) of the opening (72), and the bolt (106) is movable, parallel to the straight portion (92) of the engagement hook (91), between an unlocking position and a locking position.

3. Support device (90) according to the preceding claim, in which the bolt (106) is provided and designed such that, in the unlocking position, it is not arranged next to the straight portion (92) of the engagement hook (91) and, in the locking position, it is arranged next to the straight portion (92) of the engagement hook (91).

4. Support device (90) according to one of the preceding claims, in which the bolt (106) is provided and designed such that, in the unlocking position, it does not engage in an opening (72) on an equipment carrier (10) in which the engagement hook (91) engages, and, in the locking position, it does engage in an opening (72) on an equipment carrier (10) in which the engagement hook (91) engages.

5. Support device (90) according to Claim 1, also with a second plate (104), which is rigidly connected to the spar (100) and to the bolt (106), wherein the first plate (94) and the second plate (104) are connected to each other with a form fit in the stable position.

6. Support device (90) according to the preceding claim, also with a cover cap (108) or a bracket that can be turned over the first plate (94) and the second plate (104) in order to hold the second plate (104) relative to the first plate (94) in the stable position.

7. Equipment carrier (10) for carrying a medical appliance, a medical instrument or another payload, **characterized by** a supporting framework (20, 30, 40, 60), and an opening (72) on the supporting framework (30, 40), wherein the opening (72) is provided and designed for receiving an engagement hook (91) on a support device (90) and is a support device (90) according to one of Claims 1 to 6.

8. Equipment carrier (10) according to the preceding claim, in which a plurality of openings (72) are provided on the supporting framework (30, 40), wherein an engagement hook (91) on a support device (90) can engage alternately in one of the plurality of openings (72).

9. Equipment carrier (10) according to the preceding claim, in which the supporting framework comprises several columns (30, 40), several respective openings (72) are provided on each column (30, 40), respective openings (72) with different markings are provided on each column (30, 40) openings (72), which receive hook devices (91) of the support device (90) simultaneously, in an intended arrangement of a support device (90), have identical or corresponding markings.

## Revendications

1. Dispositif de support (90) pour la connexion amovible à une servante d'outillage (10) et pour supporter un appareil médical, un instrument médical ou une autre charge utile dans une salle de traitement médicale, comprenant : un crochet d'engagement (91) destiné à s'engager dans une ouverture (72) au niveau d'une servante d'outillage (10) ; un verrou (106) pour verrouiller une connexion entre le crochet d'engagement (91) et une ouverture (72) dans laquelle s'engage le crochet d'engagement (91), **caractérisé par** un longeron (100) pour supporter un appareil médical ou un instrument médical ou une autre charge utile, le longeron (100) pouvant être connecté au crochet d'engagement (91), le verrou (106) étant connecté rigidement au longeron (100) ; une première plaque (94) qui est connectée rigidement au crochet d'engagement (91) et qui présente un alésage (95), le verrou (106), dans la position de verrouillage, s'engageant à travers l'alésage (95) de la première plaque (94), le longeron (100), dans la position de verrouillage du verrou (106), pouvant pivoter autour d'un axe (105) s'étendant à travers le verrou (106) entre une position de libération et une position fixée, le verrou (106), dans la position de libération du longeron (100), pouvant être déplacé entre sa position de verrouillage et sa position de déverrouillage et le longeron (100) pouvant être séparé du crochet d'engagement (91), le longeron (100), dans la position fixée du manchon (100), étant connecté mécaniquement au crochet d'engagement (91) et étant prévu pour être sollicité par une charge utile.

2. Dispositif de support (90) selon la revendication précédente, dans lequel le crochet d'engagement (91) comprend une portion droite (92) pour l'engagement dans une ouverture (72) et une portion coudée (93) par rapport à la portion droite (92) pour s'engager par l'arrière avec un bord (73) de l'ouverture (72), le verrou (106) pouvant être déplacé parallèlement à la portion droite (92) du crochet d'engagement (91) entre une position déverrouillée et une position de verrouillage.

3. Dispositif de support (90) selon la revendication précédente, dans lequel, dans la position de déverrouillage, le verrou (106) est prévu et réalisé pour ne pas être disposé à côté de la portion droite (92) du crochet d'engagement (91), et pour être disposé à côté de la portion droite (92) du crochet d'engagement (91) dans la position de verrouillage.

4. Dispositif de support (90) selon l'une quelconque des revendications précédentes, dans lequel, dans la position déverrouillée, le verrou (106) est prévu et réalisé pour ne pas venir s'engager dans une ouverture (72) au niveau d'une servante d'outillage (10) dans laquelle s'engage le crochet d'engagement (91), et dans la position verrouillée de verrouillage, pour s'engager dans une ouverture (72) au niveau d'une servante d'outillage (10) dans laquelle s'engage le crochet d'engagement (91) .

5. Dispositif de support (90) selon la revendication 1, comprenant en outre : une deuxième plaque (104) qui est connectée rigidement au longeron (100) et au verrou (106), la première plaque (94) et la deuxième plaque (104) étant connectées l'une à l'autre par engagement par correspondance de formes dans la position fixée.

6. Dispositif de support (90) selon la revendication précédente, comprenant en outre : un capuchon de recouvrement (108) ou une pince qui peut être replié(e) par-dessus la première plaque (94) et la deuxième plaque (104) afin de retenir la deuxième plaque (104) par rapport à la première plaque (94) dans la position fixée.

7. Servante d'outillage (10) pour porter un appareil médical, un instrument médical ou une autre charge utile, **caractérisée par** un châssis (20, 30, 40, 60) ; une ouverture (72) au niveau du châssis (30, 40), l'ouverture (72) étant prévue et réalisée pour recevoir un crochet d'engagement (91) au niveau d'un dispositif de support (90), et un dispositif de support (90) selon l'une quelconque des revendications 1 à 6.

8. Servante d'outillage (10) selon la revendication précédente, dans laquelle une pluralité d'ouvertures (72) sont prévues sur le châssis (30, 40), un crochet d'engagement (91) pouvant s'engager au niveau d'un dispositif de support (90) et en variante dans l'une de la pluralité d'ouvertures (72).

9. Servante d'outillage (10) selon la revendication précédente, dans laquelle le châssis comprend plusieurs colonnes (30, 40), plusieurs ouvertures (72) sont à chaque fois prévues sur chaque colonne (30, 40), des ouvertures (72) au niveau de chaque colonne (30, 40) sont à chaque fois pourvues de marquages différents ouvertures (72), qui dans le cas d'un agencement prévu d'un dispositif de support (90), reçoivent simultanément des dispositifs de crochets (91) du dispositif de support (90), présentent des marquages correspondants ou identiques.
